# FASCICULE DE BREVET EUROPEEN

(11) **EP 0 910 622 B1**
(45) Date de publication et mention de la délivrance du brevet: **26.09.2012**
(21) Numéro de dépôt: 97914356.7
(22) Date de dépôt: 05.03.1997
(51) Int. Cl.: C12N 1/04

(54) **MILIEU POUR LA CONSERVATION DE MATERIEL BIOLOGIQUE**
MEDIUM ZUR KONSERVIERUNG BIOLOGISCHEN MATERIALS
MEDIUM FOR PRESERVING BIOLOGICAL MATERIALS

(30) Priorité: 12.03.1996 FR 9603074
(43) Date de publication de la demande: 28.04.1999
(73) Titulaire: Aventis Pharma S.A., 92160 Antony (FR)
(72) Inventeur: CRESPO, André, F-94490 Ormesson (FR); SORIA, Henri-Michel, F-37260 Monts (FR)
(74) Mandataire: Holtz, Béatrice
(86) Numéro de dépôt international: PCT/FR1997/000385
(87) Numéro de publication internationale: WO 1997/033975

(56) Documents cités:
- EP-A- 0 162 332
- EP-A- 0 508 496
- FR-A- 2 042 381
- VOX SANGUINIS, vol. 51, 1986, pages 81-86, XP000611822 TOMONO ET AL: "A NEW INTACT IMMUNOGLOBULIN FOR INTRAVENOUS USE STABILIZED BY CHEMICALLY MODIFIED GELATIN DERIVATIVES"

## Description

La présente invention concerne l'utilisation d'un milieu permettant la congélation de matériels biologiques tels que cellules animales et particules virales, directement injectable ou réinjectable dans un organisme. Elle concerne plus particulièrement l'utilisation d'un milieu comprenant une solution saline, de la gélatine fluide modifiée et de la sérum albumine humaine,

Aussi bien dans la thérapie cellulaire ou la thérapie génique que dans la transfusion sanguine ou la greffe de moelle osseuse l'un des principaux problèmes rencontrés est celui de la conservation de matériel biologique. Il est en effet important de pouvoir conserver le matériel biologique, dans de bonnes conditions de viabilité, pendant une période de temps suffisamment longue et compatible avec la production et le stockage à l'échelle industrielle et également pour permettre d'effectuer certains tests. La méthode de conservation la plus couramment utilisée consiste à congeler ledit matériel, Cependant, lors de la congélation de cellules, par exemple, celles-ci subissent un stress très important dû en particulier à un phénomène d'exosmose et à la formation de glace à l'intérieur de la cellule. Ces phénomènes entraînent de nombreuses lyses cellulaires aussi bien lors de la congélation que lors de la décongélation. Même les cellules non lysées peuvent voir leur capacité à se diviser ou à se différencier altérée de manière irréversible. Il est très important de pouvoir obtenir un taux de viabilité important après décongélation pour des cellules devant être injectées dans un organisme vivant (transfusion sanguine, greffe de moelle osseuse ou thérapie cellulaire par exemple), il est en effet inutile de réinjecter des cellules mortes ou abîmées. De même lorsqu'il s'agit de cellules devant être remises en culture il est tout aussi important que le taux de viabilité soit élevé. Jusqu'à présent on ajoutait dans le milieu un agent cryoprotecteur qui empêchait la lyse cellulaire. L'agent protecteur le plus utilisé et qui donne les meilleurs résultats est un conservateur non injectable, le diméthyl sulfoxyde ou DMSO. Le DMSO s'insère dans les membranes cellulaires et permet de les stabiliser. Il empêche ainsi la destruction des cellules. Un système comme celui-ci permet au moment de la décongélation d'avoir un grand nombre de cellules vivantes et viables qui sont aptes à se diviser. Mais cette méthode pose un problème majeur quand lesdites cellules doivent être introduites ou réintroduites dans un organisme. En effet le DMSO est un produit toxique pour les cellules à températures ambiante. Le DMSO perméabilise les membranes entraînant la mort de la cellule. Il doit donc être éliminé avant que l'on puisse réimplanter les cellules s'il s'agit d'une autogreffe de cellules modifiées ou les implanter s'il s'agit d'une hétérogreffe par exemple de cellules de moelle osseuse ou encore effectuer la transfusion s'il s'agit de cellules sanguines. L'élimination du DMSO se fait, après décongélation, généralement par dilution de l'échantillon dans dix volumes de milieu sans DMSO, puis centrifugation et élimination du surnageant. Cette opération est répétée plusieurs fois, jusqu'à élimination de la quasi totalité du DMSO. Ce traitement occasionne une perte de temps et, en multipliant les manipulations, augmente les risques de contaminations par des agents pathogènes extérieurs et de pertes dudit matériel biologique.

En présence de DMSO le pourcentage de cellules viables après décongélation est supérieur à soixante dix pour cent dans les conditions optimales de congélation/décongélation telles que définies plus loin. Sans DMSO ce pourcentage est inférieur a vingt pour cent. Jusqu'à présent la congélation en présence de DMSO était la méthode la plus efficace donc la plus employée.

La demanderesse s'est intéressée à un nouveau type de milieu qui permet d'éviter les manipulations subséquentes à la décongélation tout en gardant un pourcentage de cellules viables élevé. Pour cela la demanderesse a mis au point un milieu de congélation/conservation qui permet d'obtenir une viabilité élevée à la décongélation sans utiliser de DMSO ou un autre cryoconservateur cytotoxique. L'avantage d'un tel milieu vient de ce que la solution est injectable tout de suite après la décongélation sans qu'aucune manipulation ne soit nécessaire. Il devient alors possible de procéder à la décongélation directement dans la salle d'opération ce qui réduit le temps entre la décongélation et l'utilisation, ceci permet aussi de rester constamment en milieu stérile et donc de réduire au minimum les risque de contaminations extérieures.

L'invention concerne l'utilisation d'un milieu pour la congélation de cellules ou de matériel biologique contenant une information génétique autoreproductible ou reproductible dans un système biologique comprenant une solution saline, de la gélatine fluide modifiée et de la sérum albumine humaine (HSA).

Comme indiqué ci-avant, ce milieu est dépourvu de tout agent toxique et peut être administré directement à un organisme. Il peut être utilisé pour la conservation, éventuellement sous forme congelée, de matériels biologiques divers tels que virus, cellules, plaquettes, etc.

Le premier élément entrant dans la composition du milieu selon l'invention est la solution saline. La solution saline est plus particulièrement une solution isotonique avec le plasma. Les sels entrant dans la composition de cette solution peuvent varier. Avantageusement elle comprend des chlorures, tels que du chlorure de sodium, du chlorure de potassium du chlorure de calcium et/ou du chlorure de magnésium, et des lactates, tels que par exemple du lactate de sodium. Plus particulièrement, la solution saline isotonique comprend généralement du chlorure de sodium, du chlorure de potassium, du chlorure de magnésium et du lactate de sodium. Selon une autre variante, le chlorure de magnésium est remplacé par du chlorure de calcium. Dans ce cas les concentrations en sels de la solution saline sont équivalentes ou quasi équivalentes à celles d'une solution "Ringer-lactate". Une telle solution est habituellement utilisée en perfusion pour compenser une déshydratation ou une perte de liquide physiologique par exemple.

Selon un mode particulier de réalisation de l'invention, la solution saline est composée essentiellement de NaCl, MgCl2, KCl et Lactate dont les concentrations respectives finales dans le milieu sont données dans le tableau 1 ci-dessous.

**Tableau 1**

| **Sel** | **Concentration minimale (g/l)** | **Concentration maximale (g/l)** | **Exemple particulier** |
|---|---|---|---|
| NaCl | 2.0 | 9 | 5.7 |
| MgCl2 | 0.05 | 0.2 | 0.093 |
| KCl | 0.05 | 0.5 | 0.247 |
| Lactate | 0.5 | 4 | 2.25 |

La gélatine est le second élément entrant dans la composition du milieu selon l'invention. Il s'agit d'une protéine composée de divers aminoacides reliés par les groupes amino et carbonyle voisins, de manière à donner la liaison peptidique classique. Le poids moléculaire de la gélatine est caractéristique et élevé (les valeurs du poids moléculaire moyen varient d'environ 10.000 à 100.000) et il est sensiblement hétérogène pour un type ou une qualité de gélatine donnés. La gélatine est composée de molécules de types asymétrique ou en bâtonnet, résultant de l'hydrolyse des chaînes longues des résidus de polypeptide dans le tissu conjonctif blanc. L'expérience a montré que l'hydrolyse primaire du collagène a lieu à intervalles sur des sites réactifs de ces chaînes pour produire une molécule idéale de gélatine apparentée non dégradée. Ceci se poursuit à un degré variable dans une hydrolyse secondaire à intervalles statistiques sur les liaisons moins réactives de la molécule idéale de gélatine. Ceci explique comment la réaction de dégradation est responsable du schéma moléculaire hétérogène aléatoire d'un échantillon particulier de gélatine. De même, chaque protéine constituant la gélatine a un point isoélectrique défini auquel l'ionisation et, par conséquent, la réactivité physique et chimique, est minimale. Ces propriétés sont notamment la solubilité, la viscosité et la pression osmotique colloïdale. L'asymétrie de la molécule de gélatine donne donc, avec le schéma moléculaire hétérogène, les propriétés intrinsèques de formation de gel et viscosité aux solutions de gélatine préparées pour le milieu selon l'invention.

La gélatine proprement dite (stérilisée et débarrassée de substances pyrogènes et antigènes) a déjà été utilisée comme produit de remplacement du plasma sanguin, mais elle a soulevé certains problèmes, en particulier pour sa conservation, car elle se gélifie à la température ambiante. Ceci a conduit à d'autres composés dérivés de la gélatine, généralement désignés sous le terme gélatine fluide modifiée, qui permettent de pallier notamment ces inconvénients.

Parmi les gélatines fluides modifiées, on peut citer par exemple l'oxypolygélatine, obtenue par polymérisation de la gélatine avec le glyoxal et oxydation par H₂O₂. D'autres gélatines fluides modifiées sont obtenues par réaction de la gélatine (ayant de préférence une gamme de poids moléculaire d'environ 15.000 à 36.000) avec l'anhydride succinique, citraconique, itaconique, aconitique ou maléique ou le chlorure de succinyle ou de fumaryle, comme décrit dans le brevet français n° 1 291 502. Tous ces dérivés de gélatine sont compatibles avec une utilisation pharmaceutique et peuvent être introduits dans le courant sanguin directement en solution saline isotonique. Des gélatines fluides modifiées ont également été décrites dans les brevets US 2,525,753, US 2,827,419, US 3,108,995.

Plus généralement, les gélatines fluides modifiées selon l'invention sont constituées de produits d'hydrolyse du collagene modifiés chimiquement, compatibles avec une utilisation pharmaceutique. Il s'agit préférentiellement de produits ayant un poids moléculaire moyen compris entre 10 kD et 100 kD, et encore plus préférentiellement entre 15 kD et 40 kD. Ils sont préférentiellement modifiés par réaction avec un anhydride, de manière à obtenir un produit final ayant une fluidité adaptée a l'usage recherche, selon les enseignements par exemple du brevet FR 1,291,502. Il s'agit préférentiellement de l'anhydride succinique, citraconique, itaconique, aconitique ou maléique. Une gélatine fluide modifiée particulièrement avantageuse est constituée du produit d'hydrolyse du collagène ayant un poids moléculaire moyen compris entre 15 kD et 40 kD, modifié par réaction avec l'anhydride succinique. Les gélatines fluides modifiées selon l'invention peuvent être préparées par les techniques de l'homme de l'art, notamment décrites dans les brevets cités ci-avant.

Le troisième élément entrant dans la composition du milieu selon l'invention est la sérum albumine humaine. La sérum-albumine humaine (SAH) est une protéine monomérique non glycosylée de 585 acides aminés, d'un poids moléculaire de 66 KD. Sa structure globulaire est maintenue par 17 ponts disulfures qui créent une série séquentielle de 9 boucles doubles (Brown, J.R., "Albtunin Structure, Function and Uses", Rosenoer, V.M. et al. (eds.) Pergamon Press, Oxford, (1977) 27-51). Les gènes codant pour la SAH sont connus pour être hautement polymorphes, et plus de 30 variants génétiques apparemment différents ont été repérés par analyse électrophorétique dans des conditions variées (Weitkamp, L.R. et al., Ann. Hum. Genet. 37 (1973) 219-226). Le gène de la SAH est coupé en 15 exons par 14 séquences introniques et comprend 16 961 nucléotides, du site de "capping" supposé jusqu'au premier site d'addition de poly(A).

L'albumine humaine est synthétisée dans les hépatocytes du foie, puis sécrétée dans le flux sanguin. Cette synthèse conduit dans un premier temps à un précurseur, la prépro-SAH, qui contient une séquence signal de 18 acides aminés dirigeant le polypeptide naissant dans la voie de sécrétion.

La SAH est la protéine la plus abondante du sang, avec une concentration d'environ 40 g par litre de sérum. Il y a donc environ 160 g d'albumine circulante dans le corps humain à tout moment. Le rôle le plus important de la SAH est de maintenir une osmolarité normale du flux sanguin. Elle présente également une capacité exceptionnelle de liaison pour diverses substances et joue un rôle aussi bien da le transport endogène de molécules hydrophobes (tels que les stéroïdes et les sels biliaires) que dans celui de différentes substances thérapeutiques qui peuvent ainsi être transportées à leurs sites d'action respectifs. De plus, la SAH a été récemment impliquée dans le catabolisme des prostaglandines.

La SAH utilisée dans le cadre de la présente invention peut être soit d'origine naturelle (SAH purifiée) soit d'origine recombinante (rSAH).

A cet égard, la SAH naturelle est généralement produite par purification à partir de matériel biologique d'origine humaine. En particulier, elle est obtenue par les techniques classiques de fractionnement du plasma provenant de dons de sang (Cohn et al., J. Am. Chem. Soc. 68 (1946) 459 pp), ou par extraction à partir du placenta humain, selon la technique décrite par J. Liautaud et al. (13ème Congès International d'IABS, Budapest; A: "Purification of proteins. Development of biological standard", Karger (ed.), Bale, 27 (1973) 107 pp). De préférence l'albumine purifiée utilisée dans le cadre de la présente invention est une albumine plasmatique. Tout particulièrement on peut utiliser une solution d'albumine plasmatique commerciale.

Le développement du génie génétique et des nouvelles techniques d'extraction et de purification a ouvert la possibilité d'obtenir, à un prix de revient plus faible, des produits améliorés de plus haute pureté, de meilleure stabilité et sans risque de contamination virale (par exemple hépatite B et SIDA). Compte tenu de l'importance du marché de la SAH, la possibilité de produire cette protéine par voie recombinante a été largement étudiée. Ainsi, de nombreux systèmes d'expression ont été étudiés pour la préparation de la SAH recombinante.

Plus particulièrement, en ce qui concerne les hôtes bactériens, les premières expériences de génie génétique ont utilisé la bactérie E.coli comme organisme hôte. Ainsi, les brevets européens EP 236 210, EP 200 590, EP 198 745, ou EP 1 929 décrivent des procédés de production de la SAH chez E.coli en utilisant différents vecteurs d'expression, différents promoteurs transcriptionnels, et différents signaux de sécrétion. Par la suite, des travaux concernant la sécrétion de la SAH dans Bacillus subtilis ont également été réalisés (Saunders et al., J. Bacteriol. 169 (1987) 2917). En ce qui concerne les hôtes eucaryotes, des procédés pour la production de la SAH ont été mis au point en utilisant des levures comme organisme hôte. Ainsi, la production de la SAH sous le contrôle du promoteur de la chélatine a pu être mise en évidence dans S.cerevisiae (Etcheverry et al., Bio/Technology 4 (1986) 726). La production de la SAH a également été mentionnée dans la levure de brasserie lors de la fabrication de la bière, en utilisant un procédé post-fermentaire (EP 201 239). Plus récemment, la demande de brevet EP 361 991 décrit un système particulièrement performant utilisant la lévure Kluyveromyces comme organisme hôte, transformée avec des vecteurs dérivés du plasmide pKD1. Des niveaux particulièrement élevés de SAH sécrétée dans le milieu de culture ont pu être obtenus avec ce système. Enfin, la production de SAH recombinante a également été décrite dans Pichia pastoris, (EP 344 459). En outre, la purification de la SAH a également fait l'objet de nombreuses études (EP 319 067).

On utilise avantageusement une SAH, recombinante ou naturelle, respectant certains critères de qualité (homogénéité, pureté, stabilité). Ainsi, la pharmacopée fixe un certain nombre de paramètres pour les solutions d'albumine plasmatique, à savoir une valeur de pH, une teneur en protéines, une teneur en polymères et agrégats, une teneur en phosphatase alcaline et une certaine composition en protéines. Elle impose de plus une certaine absorbance, la conformité à un test de stérilité, à un essai de pyrogènes et de toxicité (voir "Albumini humani solutio" pharmacopée Européenne (1984) 255). Il est particulièrement preféré d'utiliser une albumine répondant a ces critères, bien que cela ne soit pas indispensable.

Avantageusement, les compositions selon l'invention comprennent une albumine humaine plasmatique purifiée ou un albumine humaine recombinante, préférentiellement produite chez un hôte eucaryote. En outre, le terme SAH comprend, au sens de l'invention, tout variant naturel de l'albumine humaine, résultant du polymorphisme de cette protéine. Il est également possible d'utiliser un équivalent de la SAH, c'est-à-dire tout dérivé de la SAH conservant les propriétés de la SAH. Ces dérivés peuvent être notamment des fragments N-terminaux de la SAH.

Les milieux utilisés selon l'invention peuvent être préparés de différentes façons. Les différents composants peuvent être mélanges ensemble, puis le matériel biologique ajouté au melange. Il est egalement possible de melanger un ou deux des composants au materiel biologique, puis d'ajouter ensuite le ou les deux derniers composants. Préférentiellement, un milieu comprenant les trois composants est préparé, auquel est ensuite ajouté le matériel biologique. La préparation du milieu et l'ajout de matériel biologique sont réalises dans des conditions stériles. Selon un mode particulier de réalisation, la gélatine fluide modifiée est ajoutée à une solution saline, puis la SAH est ajoutée au milieu. A cet égard, un mode préféré de mise en oeuvre consiste à utiliser un mélange particulier ayant la même composition qu'un succedané de plasma sanguin, le Plasmion (brevet FR 2.042.381), auquel la SAH est ensuite ajoutée. Le plasmion est une solution commerciale composée d'une solution saline et de gélatine fluide modifiée. Sa composition est la suivante:
- Gélatine fluide modifiée 30 g/l
- Chlorure de sodium 5.382 g/l
- Chlorure de magnésium 0.143 g/L
- Chlorure de potassium 0.373 g/l
- Lactate de sodium 3.360 g/l
- Eau pour préparation injectable q.s.p. 1000 ml

Le plasmion est usuellement utilisé comme soluté de remplissage vasculaire dans le rétablissement du volume sanguin circulant, ou pour une hémodilution avec baisse de la viscosité sanguine et une amélioration de la microcirculation, ou encore pour le rétablissement de l'équilibre ionique et la prévention de l'acidose. Un objet préféré de la présente invention concerne l'utilisation d'un milieu permettant la congélation de matériel biologique dans lequel le plasmion tient lieu de mélange de solution saline et de gélatine fluide modifiée. Un tel milieu comprend du plasmion et de la sérum albumine humaine.

Les proportions respectives des composants des milieu utilisés selon l'invention peuvent être adaptées par l'homme du métier en fonction du matériel biologique considéré. Comme illustré dans les exemples, bien que certaines gammes de concentration soient préférées, les proportions peuvent être modifiés. A cet égard, les fourchettes préférées de concentrations en sels ont été presentées dans le tableau 1 ci-avant. Concernant la gélatine et la sérum albumine, elles varient préférentiellement dans un rapport de poids albumine/gélatine compris entre 0.5 et 100. Ce rapport est plus préférentiellement compris entre 0.5 et 60, et, de manière particulièrement préférée, entre 3 et 15. A titre d'exemples particuliers, on peut mentionner des rapports de 0.74, 1.66, 3.3, 6.66, 13.4, 26.66 et 60. Ces différents rapports correspondent à des milieux selon l'invention contenant de 10% a 90% en volume d'une solution commerciale de plasmion et de 90% a 10% en volume d'une solution de sérum albumine humaine à 20%. Différentes compositions sont représentées à titre illustratif dans le tableau 2 ci-apres.

**Tableau 2**

| Plasmion %v | Gélatine Poids (g/l) | SAH 20% %v | SAH Poids (g/l) | Rapport empirique SAH/ Gélatine |
|---|---|---|---|---|
| 10 | 3 | 90 | 180 | 60 |
| 90 | 27 | 10 | 20 | 0.74 |
| 80 | 24 | 20 | 40 | 1.66 |
| 20 | 6 | 80 | 160 | 26.66 |
| 50 | 15 | 50 | 100 | 6.66 |
| 67 | 20 | 33 | 66 | 3.3 |
| 33 | 10 | 67 | 134 | 13.4 |

D'une manière générale, le milieu utilisé selon l'invention contient de 10 à 90% en volume de plasmion respectivement de solution de sérum albumine humaine à 20%. Les modes de réalisation particuliers sont des milieux comprenant entre 20 et 80% de plasmion respectivement de solution de sérum albumine humaine à 20%, plus préférentiellement entre 33 et 67% de plasmion respectivement de solution de sérum albumine humaine à 20%. Un autre mode de réalisation particulier de l'invention consiste en un mélange de plasmion et de solution de sérum albumine humaine à 20% dans un rapport volume à volume de 50/50. Avantageusement, le rapport en poids SAH/gélatine est compris entre 2 et 7. Des résultats particulièrement remarquables ont été obtenus avec un rapport de 3 environ.

Par ailleurs, des composants supplémentaires peuvent être ajoutés aux milieux utilisés selon l'invention. En particulier, des agents de stabilité cellulaire biocompatibles peuvent être introduits, tels que par exemple des composés de la famille du glycerol (glycine, glycerol, saccharose, glucose, etc). Ces composés sont présents dans les milieux de l'invention dans des quantites inferieures a 5% en poids. Préférentiellement, les milieux selon l'invention comprennent entre 0.5 et 5% en poids de glycine ou glycerol.

Les milieux utilisés selon l'invention servent à la congélation de matériel biologique. On entend généralement par matériel biologique tout matériel contenant une information génétique, qui est autoreproductible ou reproductible dans un système biologique. Le dit matériel biologique peut être constitué plus particulièrement par des cellules ou des particules virales ou encore les deux. Parmi les cellules pouvant être congelées on peut citer par exemple les cellules sanguines, les cellules de la moelle osseuse, les cellules productrices de particules virales (des lignées de "packaging"), ou les cellules génétiquement modifiées.

Les particules virales plus particulièrement concernées par la présente invention sont celles pouvant être utilisées en thérapie génique. Un grand nombre de virus peuvent voir leur génome modifié d'une part pour qu'ils perdent leur faculté de se multiplier tout en gardant leur pouvoir d'infection, d'autre part pour insérer dans leur génome une séquence d'acide nucléique d'intérêt thérapeutique qui sera exprimée dans les cellules infectées. Parmi ces virus, on peut citer plus particulièrement les adenovirus, les AAV, les retrovirus, les virus de l'herpes, etc.

Les adénovirus sont parmi les virus les plus employés. Différents sérotypes d'adénovirus, dont la structure et les propriétés varient quelque peu, ont été caractérisés. Parmi ces sérotypes, on préfère utiliser dans le cadre de la thérapie génique les adénovirus humains de type 2 ou 5 (Ad 2 ou Ad 5) ou les adénovirus d'origine animale (voir demande W094/26914). Parmi les adénovirus d'origine animale utilisables on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise des adénovirus d'origine humaine ou canine ou mixte.

Les adénovirus défectifs comprennent les ITR, une séquence permettant l'encapsidation et un acide nucléique d'intérêt. Dans le génome de ces adénovirus, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697) et L5 (WO95/02697). Il peut également comprendre une délétion dans région E1 au niveau de laquelle sont insérés la région E4 et l'acide nucléique d'intérêt thérapeutique (Cf FR94 13355).

Les adénovirus recombinants défectifs peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN d'intérêt. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison.

Ensuite, les adénovirus qui se sont multipliés sont récupérés et purifiés selon les techniques classiques de biologie moléculaire, généralement sur gradient de chlorure de cesium.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs peuvent être préparés par co-transfection, dans une lignée cellulaire infectée par un virus auxiliaire humain (par exemple un adénovirus), d'un plasmide contenant une séquence nucléique d'intérêt bordée de deux régions répétées inversées (ITR) d'AAV, et d'un plasmide portant les gènes d'encapsidation (gènes rep et cap) d'AAV. Une lignée cellulaire utilisable est par exemple la lignée 293. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants défectifs comportant un acide nucléique d'intérêt thérapeutique, un plasmide comportant notamment les LTR, la séquence d'encapsidation et ledit acide nucléique est construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env (Cf ci-dessous).

Les particules virales, généralement recombinantes (contenant un acide nucléique d'intérêt) et défectives (incapables de réplication autonome) peuvent être conservées dans un milieu selon l'invention. Généralement, les particulies virales (adeno, AAV, retro, etc) sont utilisées sous forme purifiée, puis conditionnée dans un milieu selon l'invention en vue de leur conservation, préférentiellement sous forme congelée. Généralement, 10⁴ a 10¹⁴ particules virales peuvent être conditionnées pour 1 ml de milieu selon l'invention, dans un containeur stérile. Préférentiellement, on utilise 10⁵ a 10¹⁰ particules virales par ml de milieu et, encore plus préféentiellement, 10⁸ ou 10⁹.

Les lignée cellulaires dites lignées d'encaspsidation (ou de packaging) dont il a déjà été question ci-dessus sont des lignées cellulaires utilisées pour la production de virus recombinant défectifs, in vitro ou in vivo (apres implantation). On peut citer à titre d'exemple de lignée pour la production d'adénovirus, la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697. On peut également citer des lignées d'encapsidation qui ont été décrites pour la production de rétrovirus ou d'herpés virus, et notamment la lignée PA317 (US4,861,719); la lignée PsiCRIP (WO90/02806) et la lignée GP+envAm-12 (WO89/07150) ou des lignees dérivees productrices de retrovirus recombinants, telles que la lignée M11 (WO94/13824). Comme illustré dans les exemples, le milieu de l'invention est particulièrement adapté à la conservation de cellules productrices de particules virales.

Un autre type de matériel biologique qui peut être conservé avantageusement dans un milieu selon l'invention est constitué de cellules génétiquement modifiées. Les cellules génétiquement modifiées sont des cellules, destinées notamment à la thérapie génique, dans lesquelles une séquence d'acide nucléique d'intérêt a été introduite. Depuis quelques années le nombre de ces cellules n'a cessé de croître et on peut citer à titre d'exemples les cellules souches hématopoiétïques (WO88/08450, WO93/20195, WO93/09815, WO93/11230), les cellules endothéliales (WO89/05345, WO90/06757, WO92/09222), les myoblastes (WO93/03768, WO93/24151, WO94/01129), les fibroblastes (US 5,219,740, WO89/02468, WO93/07906), les hépatocytes (WO89/07136, WO92/12242, WO93/03142), les astrocytes (WO94/01135), les neuroblastes (WO94/10292, WO94/16059), les kératinocytes (WO94/11011), les macrophages (FR93/10222). Ces cellules possèdent généralement la capacité de produire un produit d'intérêt thérapeutique et peuvent être implantées in vivo. Parmi les cellules sanguines, on peut citer les erythrocytes, les granulocytes neutrophiles, basophiles et éosinophiles, les lymphocytes B et T, notamment les lymphocytes CD4, les lymphocytes cytotoxiques (CD8 CTL), les lymphocytes infiltrant les tumeurs (TIL) et les LAK, les monocytes et les macrophages, les cellules dendritiques, les mégacaryocytes et les plaquettes. Ces cellules peuvent également être modifiées génétiquement pour acquérir de nouvelles propriétés thérapeutiques.

Le milieu peut, selon l'invention, en outre être utilisé pour la conservation de cultures primaires de cellules, et de cellules tumorales ou biopsies de tumeurs. Ce type de matériel est actuellement en cours d'étude dans des essais cliniques de thérapie immuniadoptive dans lesquels une tumeur est prelevée d'un patient, traitée par différents agents immunopotentiateurs (introduction de matériel génétique exprimant des lymphokines ou des antigènes tumoraux) puis réadministrée au patient. L'une des étapes délicates réside dans la conservation des cellules prélevées du patient ou modifiée avant réinfusion Le milieu permet, selon l'invention, avantageusement une bonne conservation de ces matériels avec une viabilité élevée.

L'utilisation d'un milieu selon l'invention permet de conserver ces cellules et de les injecter directement dans un organisme, sans étape de centrifugation ou lavage, avec une bonne viabilité et sans affecter leur capacité à produire des protéines thérapeutiques ou des virus, le cas échéant.

A cet égard, la présente invention concerne également des préparations contenant du milieu de conservation selon l'invention et du matériel biologique, ainsi qu'un procédé de stockage de matériel biologique. Ledit matériel biologique peut être conditionné directement dans un milieu selon l'invention. S'agissant de cellules, celles-ci sont avantageusement préalablement débarassées de leur milieu de culture (par exemple centrifugées, récoltées et lavées dans une solution tampon), avant d'être conditionnées dans un milieu selon l'invention. S'agissant de cellules prolifératives, elles sont préférentiellement utilisées à sous-confluence, en phase exponentielle de croissance. Comme indiqué dans les exemples, c'est dans ces conditions que les meilleurs résultats de viabilité peuvent être obtenus. Il est possible toutefois d'utiliser des cellules à confluence ou post-confluence. Généralement, 10⁵-10⁹ cellules sont conditionnées par ml de milieu et, plus préférentiellement, de 10⁶-10⁸. Dans le cas de cellules adhérentes, les cellules sont préalablement décollées par traitement classique, mises en suspension, puis conditionnées dans un milieu de l'invention. Le traitement utilisé pour décoller les cellules peut être un traitement enzymatique (trypsine par exemple), chimique (detergent) ou mécanique. Dans le cas d'un traitement chimique ou enzymatique, les cellules sont ensuite centrifugées et lavées pour éliminer l'enzyme ou le détergent, avant la congélation. Généralement, la viabilité des cellules est controlée avant la congélation, ainsi que leur stérilité. S'agissant de virus, ceux-ci sont préalablement purifiés comme indiqué ci-avant (centrifugation sur gradient de chlorure de cesium par exemple, chromatographies, etc). Ils peuvent être conditionnés à raison de 10⁴ a 10¹⁴ particules par ml, de préférence 10⁵ a 10¹⁰. Le matériel biologique peut ensuite être conditionné dans du milieu selon l'invention, dans un containeur approprié. Il peut s'agir d'une ampoule, d'un tube, notamment d'un cryotube, d'un sac, d'un flacon, d'une flasque, etc. Le containeur est préalablement stérilisé et les opérations de conditionnement sont réalisées en conditions stériles.

Un milieu selon l'invention permet la congélation et la décongélation de matériel biologique dans des conditions de viabilité élevée. Les milieux selon l'invention peuvent en particulier permettre la congélation de matériel biologique à des températures comprises entre -200 et -4 degres Celsius. Le matériel peut être conservé notamment dans l'azote liquide ou à des températures supérieures, pendant une durée suffisamment longue pour assurer la conservation d'un stock industriel (jusqu'à une année par exemple). Le pourcentage de cellules viables après décongélation se définit comme le nombre de cellules vivantes divisé par le nombre de cellules total multiplié par cent. Ce pourcentage de viabilité dans un milieu selon l'invention est avantageusement supérieur à 50%. Préférentiellement ce pourcentage est supérieur à 60%. D'une manière tout à fait préférentielle, ce pourcentage est supérieur à 70%.

D'autres avantages de la présente invention apparaîtront à la lecture des exemples qui suivent, qui doivent être considérés comme illustratifs et non limitatifs.

### MATERIEL ET METHODES

### Test de Viabilité Cellulaire

La viabilité des cellules a été déterminée par la technique au bleu Trypan. Le bleu trypan est un colorant qui pénètre uniquement dans les cellules mortes. Lorsqu'on observe le quadrillage très régulier d'une cellule de Malassez ou d'une Kova slide, les cellules qui se trouvent dans les petits carrés de l'ensemble du quadrillage sont comprises dans un volume très précis : 1µl. Ce sont ces cellules que l'on compte, en respectant les règles suivantes :
- Les cellules qui se trouvent sur les limites extérieures du quadrillage ne sont comptées que si elles sont sur les bords supérieur et gauche (vert), ou inférieur et droit (rouge);
- Pour que le comptage soit significatif compter deux chambres de comptage et faire la moyenne.

Le nombre de cellules mortes correspond au nombre de cellules bleues. Le nombre de cellules viables correspond nombre au de cellules blanches réfringentes. La viabilitée est expriméee par le rapport du nombre de cellules viables sur le nombre de cellules viables plus le nombre de cellules mortes.

Il est entendu que d'autres techniques de comptage ou détermination de la viabilité cellulaire peuvent être utilisées.

### Cellules utilisées

Les cellules utilisées dans les exemples sont des fibroblastes génétiquement modifiés, capables de produire des particules virales. Il s'agit plus précisement de la lignée cellulaire M11 déposée a la Collection Nationale de Culture de Microorganismes, sous la référence I-1278. Cette lignée dérive des cellules PsiCRIP.

Des cellules équivalentes sont par exemple les cellules de la lignée Am12 (WO89/07150). Il est entendu que le procédé décrit est transposable directement à d'autres cellules, notamment d'origine humaine, qu'il s'agisse de cultures primaires ou de lignées établies.

### Albumine

L'albumine utilisée dans le cadre des exemples est commercialisée par la société Armour sous la référence Albumin A ou Albumine 20% plasmatique humaine. Il est entendu que toute autre source d'albumine peut être employée.

### Plasmion

Le plasmion est d'origine commerciale (Roger Bellon, France).

### EXEMPLES:

### Exemple 1 : Protocole de congélation du matériel biologique.

Pour la congélation de cellules, il est préférable d'utiliser des cellules subconfluentes en phase exponentielle de croissance. Par ailleurs, pour améliorer la survie du matériel biologique, la répartition et la congélation en cryotubes de la suspension cellulaire après mise en contact avec le milieu de congélation selon l'invention sont effectuées aussi rapidement que possible. Enfin, les manipulations sont effectuées dans des conditions stériles (par exemple sous une hotte à flux laminaire).

Cet exemple décrit plus particulièrement un protocole pour la congélation de cellules (cellules génétiquement modifiées, cellules sanguines, cellules productrices de virus, etc). Il est entendu que ce protocole peut être adapté par l'homme du métier à la congélation de virus ou autre matériel.

Les flacons de culture contenant les cellules à congeler sont sortis de l'incubateur, et placés sous un microscope, pour contrôler l'aspect de la culture. Si un ou plusieurs flacons présentent un aspect non conforme (des cellules décrochées, confluentes, une culture trouble, des cellules non refringentes, un flacon dérérioré, etc) il n'est pas utilisé pour la congelétion.

Les flacons conformes sont ensuite transférés sous une hotte à flux laminaire. Lorsque les cellules sont des cellules adhérentes, elles sont traitées pour les decoller et/ou dissoudre les aggrégats. Pour cela, il est possible d'utiliser la trypsine ou tout autre milieu de dissociation (detergent, etc). Les suspensions cellulaires sont ensuite rassemblées dans un flacon stérile et homogénéisées doucement par pipetage. Un aliquot de 1ml est prélevé pour le comptage. Si la viabilité de la culture est inférieure à 80%, la suspension cellulaire est rejetée.

La suspension est ensuite répartie, de façon égale (par exemple à la pipette), dans un nombre pair de tubes à centrifuger, puis centrifugée 10 minutes à 400 G.

Les containers de congélation à l'alcool sont passés sous la hotte, ainsi qu'un flacon de milieu de congélation à +4°c. Le volume de milieu de congélation nécessaire pour obtenir une concentration cellulaire de 10⁷ cellules viables/ml est alors placé dans un flacon stérile.

Après centrifugation, le surnageant est éliminé, puis les culots sont repris avec des aliquots de milieu de congélation froid. Les suspensions sont homogénéisées et reprises dans le flacon contenant le milieu de congélation, et homogénéisées à nouveau. Un échantillon est préléve pour test de stérilité.

La suspension cellulaire est alors répartie dans les cryotubes, qui sont ensuite placés aussitôt dans le container de congélation à alcool. Les containers sont transférés 1 heure à +4°c, puis placés dans une enceinte à -80°c pendant au moins 12H, de préférence 24H. 24H au moins après congélation, les ampoules sont sorties des containers à alcool et stockées dans un container à azote liquide.

### Exemple 2 : Protocole de décongélation du matériel biologique.

### A. Produits utilisés

- Bleu Trypan,
- Tampon Phosphate (PBS) stérile, pH 7.2,
- Ethanol 70%,
- Milieu de culture
- Sérum de Veau Fetal (SFV)
- Pot d'eau sterile a 37° +/- 0.5°C(50 à 200 ml)

### B. Protocole

La décongélation est avantageusement réalisée en conditions stérile, par exemple sous une hotte.

Dans un tube stérile de 50ml, préparer un volume de milieu de culture a 20 % de SVF correspondant au 9/10 du volume du lot (de l'ampoule) à décongeler (milieu de décongélation). Ainsi, 7 ml de milieu de culture plus 2 ml de SVF sont prepares dans le cas d'une ampoule de 1 ml.

L'ampoule à décongeler est plongée dans le pot d'eau stérile à 37 degrés Celsius environ, sans l'immerger, sous agitation douce jusqu'à disparition du glaçon. Essuyer rapidement l'ampoule à l'éthanol 70%. Pipeter le contenu de l'ampoule et le transférer dans le tube contenant le milieu de décongélation puis, sans changer de pipette, réaspirer un volume identique et rincer une fois l'ampoule. Homogénéiser doucement la suspension et prélever 1 ml dans un tube stérile pour comptage. En fonction du résultat de comptage, la suspension cellulaire est transféree dans un flacon de culture en diluant avec la quantité de milieu de décongélation nécessaire pour obtenir une concentration cellulaire initiale comprise entre 2.0 et 3.0 10⁵ cellules viables par millilitre de culture.

Pour permettre de suivre dans le temps la viabilité cellulaire après décongélation, le flacon de culture est alors placé dans l'incubateur à CO2 à 37°C+/- 0.5 ; ou dans le cas de milieu tamponé HERPES dans une chambre chaude a 37°C+/- 0.5.

Dans le cas d'applications thérapeutiques, seul un aliquot du contenu de l'ampoule est utilisé pour le test de viabilité cellulaire et de stérilité. En cas de conformité, le contenu de l'ampoule décongelée peut être alors directement injecté.

### Exemple 3 : Etude du pourcentage de viabilité après congélation et décongélation de cellules productrices de rétrovirus dans différents milieux selon l'invention.

Les cellules utilisées dans cet exemple sont les cellules de la lignée M11 productrices de rétrovirus recombinants. Les cellules sont utilisées en phase exponentielle de croissance. Les cellules ont été congelées selon le protocole général décrit dans l'exemple 1. Pour cela, les cellules ont été décollées de la boîte avec du milieu de dissociation (PBS 1X; EDTA 0.02%) : 3ml pour une boite T75; 5 ml pour une boite T160 ou T225. Les cellules sont incubées 5 min dans ce milieu sous agitation douce, puis récupérées dans un tube Falcon. Un aliquot est utilisé pour compter les cellules. La suspension est ensuite centrifugée 5 min a 1000 rpm a 20 °C. Les cellules sont alors réparties dans des ampoules de congélation contenant 1 ml de milieu selon l'invention, à raison de 10⁷ cellules par ml. Les différents milieux testés sont décrits ci-après (tableau 3). Les ampoules sont mises à congeler selon le protocole décrit dans l'exemple 1. A une date déterminée, les ampoules sont décongelées selon le protocole décrit dans l'exemple 2, et la suspension cellulaire est transférée dans des flasks de culture 37°C, 5% CO2. La viabilité cellulaire est déterminée comme indiqué dans les Matériels et Méthodes. Les résultats obtenus sont présentés dans les tableaux 4-10 suivants. Ils montrent clairement un pourcentage de viabilité élevé en présence d'un milieu de l'invention. Ainsi, plus de 70% de cellules sont viables pour certains milieux de l'invention. D'une manière générale, la viabilité cellulaire est toujours supérieure a 50%. Il est à noter qu'aucun composant seul des milieux de l'invention ne permet d'obtenir une stabilité supérieure à 25%.

Un contrôle de viabilité et de reprise en culture a été effectué sur des lots après 7 mois de congélation. Ainsi, 4 ampoules de cellules congelées dans une solution de Plasmion 67% / HSA 33% (voir exemple 1) ont été décongelées après 7 mois, selon le protocole de l'exemple 2. Les quatre ampoules ont été mises en culture dans un flacon de 75 cm2. La viabilité et la reprise enculture ont été déterminées et sont reportées dans le Tableau 11.

Ces résultats montrent un pourcentage de viabilité élevé, et une bonne reprise en culture des cellules. Les cellules présentent une adhérence normale et un aspect réfringent normal. Les passages P1 et P2 (175 cm2) se sont effectués dans des conditions normales de culture.

### Exemple 4 : Amélioration de la reprise en culture

Afin d'étudier la reprise en culture des cellules traitées selon l'invention, des tests ont été effectués sur différents milieux supplémentés par des agents de stabilité cellulaire, et en particulier par différentes concentrations de glycérol.

### 4.1. Préparation des milieux:

Une est préparée selon le protocole décrit ci-avant, puis le glycérol est ensuite ajouté aux concentrations indiquées dans le tableau ci-dessous, ou, à titre de témoin, du DMSO. Brièvement, pour un volume de 10ml d'un milieu de congélation à 67% de plasmion, 33% de HSA et 2,5% de glycerol, les concentrations en volumes des composants sont :
milieu HSA/plasmion : 9,75 ml
Glycerol : 0.25ml soit 0,321g de glycérol
10ml de milieu HSA/Plasmion → 6,7ml de plasmion
9,75ml de milieu HSA/Plasmion → 6,53ml de plasmion
10ml de milieu HSA/Plasmion → 3,3ml de HSA
9,75ml de milieu HSA/Plasmion → 3,21 ml de HS

**Tableau 1.2 : Composition des milieux supplémentés**

| MILIEUX Q: 10ml | PLASMION (ml) | HSA (ml) | DMSO (ml) | GLYCEROL (ml) |
|---|---|---|---|---|
| HP+5%DMSO | 6,3 | 3,13 | 0,5 | |
| HP+2,5%DMSO | 6,53 | 3,21 | 0,25 | |
| HP+1%DMSO | 6,63 | 3,26 | 0,1 | |
| HP+10%GLYCEROL | 6,03 | 2,97 | | 1 (1.25g) |
| HP+5%GLYCEROL | 6,3 | 3,13 | | 0,5 (0.62.5g) |
| HP+2,5%GLYCEROL | 6,53 | 3,21 | | 0.25 (0.321g) |
| HP+1%GLYCEROL | 6,63 | 3,26 | | 0.1 (0.125g) |

### 4.2. Etude de la viabilité cellulaire

La congélation a été effectuée dans les conditions décrites dans l'exemple 1. Les résultats obtenus après décongélation au jour 7 sont présentés dans le tableau 13. Ils montrent que, en présence de glycérol dans une proportion inférieure à environ 5%, la viabilité cellulaire moyenne observée après décongélation est supérieure à 70%. En particulier, une viabilité moyenne de 84% est observée en présence de 1% de glycérol.

### 4.3. Etude de la reprise en culture

La reprise en culture est une variable caractérisant l'état de cellules prolifératives après décongélation. Elle est estimée dans cet exemple par le temps nécessaire aux cellules pour atteindre la confluence. Pour déterminer ce paramètre, après décongélation, la concentration cellulaire est ajustée entre 2.510⁵ et 3,510⁵ C/ml, puis les cellules sont maintenues en culture. Les résultats obtenus sont présentés dans le tableau 14. Ils montrent que la reprise en culture se fait plus rapidement lorsque les cellules ont été conservées dans un milieu supplémenté de glycérol (3,5 à 4 jours) que dans un milieu sans glycérol (4 à 5 jours). Ces résultats montrent également que l'agent de stabilité cellulaire est avantageusement introduit à raison de 0.5 à 2,5%.

### Exemple 5 : Essais de congélation programmée

Des essais de congélation programmée ont été réalisés afin de déterminer si un meilleur contrôle des paramètres de congélation pouvait influencer la qualité du matériel biologique. En particulier, un protocole de congélation permettant d'éviter la surfusion du milieu a été testé. Pour ce faire, des essais de congélation assistés par un congélateur à azote liquide Kryosave planer de chez Flobio ont été effectués, en comparaison avec des essais de congélation à l'alcool (isopropanol) selon l'exemple 1. La congélation est effectuée dans un milieu de congélation à 67% de plasmion, 33% de HSA et 2,5% ou 1% de glycerol. L'intérêt de ce système est de réaliser une congélation rapide des cellules (ou du matériel), et une descente en température des cellules et du milieu controlée, ce qui permet d'éviter le point de fusion de tout milieu congelé. Pour cela, le point de fusion est préalablement déterminé en regardant, lors d'une congélation à l'alcool, sa situation en température et dans le temps. Dans cet exemple, la température a été abaissée à -40°C pendant 10 minutes à partir de -8°C, car le point de fusion pour le matériel utilisé se trouvait à ce niveau et le milieu remontait vers 0°C. Après décongélation au jour 7, la viabilité cellulaire et la reprise en culture au bout de 24 heures sont déterminées. Les résultats obtenus sont présentés dans les tableaux 15 et 16.

Ces résultats montrent clairement une viabilité cellulaire élevée, dans tous les schémas et milieux de congélation de l'invention testés. En outre, ils montrent une bonne reprise en culture des cellules, en particulier pour des milieux comportant 1% de glycérol. Dans ce cas en effet, un rendement supérieur à 70% est observé, 24 heures après décongélation. Par ailleurs, dans le cas d'une congélation programmée contrôlée (kryosave), la nappe cellulaire observée au bout de 24 heures est belle et un nombre très limité de cellules mortes apparait.

**Tableau 3 : Composition et préparation des milieux**

| Milieu | Solution SAH 20% | Plasmion | g/l SAH | g/l Gélatine | Rapport poids S/G | Préparation (10 ml) |
|---|---|---|---|---|---|---|
| 10H/90P | 10%v | 90%v | 20 | 27 | 0.740 | 1ml SAH 9ml P |
| 20H/80P | 20%v | 80%v | 40 | 27 | 1.666 | 2ml SAH 8ml P |
| 30H/70P | 30%v | 70%v | 60 | 21 | 2.857 | 3ml SAH 7ml P |
| 40H/60P | 40%v | 60%v | 80 | 18 | 4.444 | 4ml SAH 6ml P |
| 50H/50P | 50%v | 50%v | 100 | 15 | 6.666 | 5ml SAH 5ml P |
| 60H/40P | 60%v | 40%v | 120 | 12 | 10 | 6ml SAH 4ml P |
| 70H/30P | 70%v | 30%v | 140 | 9 | 15.555 | 7ml SAH 3ml |
| 80H/20P | 80%v | 20%v | 160 | 6 | 26.666 | 8ml SAN 2ml P |
| 90H/10P | 90%v | 10%v | 180 | 3 | 60 | 9ml SAH 1ml P |

**Tableau 4 : Expérience #1 - Décongélation au jour 5**

| **Milieu de Congélation** | **% de viabilité à la décongélation** | **Nombre de cellules par ampoule (*10⁷)** | **Culture** |
|---|---|---|---|
| 67H/33P | 50.8 | 0.975 | Oui |
| 50H/50P | 63.2 | 0.51 | Oui |
| 33H/67P | 71.4 | 0.42 | Oui |
| H/P + Glycine 5% | 49.4 | 0.51 | Oui |
| H/P + Glycine 1% | 54.8 | 0.54 | Oui |

**Tableau 5 : Expérience #1 - Décongélation au jour 6**

| Milieu de **Congélation** | % de viabilité à la **décongélation** | Nombre de cellules **par ampoule (*10⁷)** | Culture |
|---|---|---|---|
| 67H/33P | 76.2 | 0.69 | Oui |
| 50H/50P | 65.9 | 0.69 | Oui |
| 33H/67P | 55.1 | 0.76 | Oui |
| H/P + Glycine 5% | 63.2 | 0.21 | Oui |
| H/P + Glycine 1% | 55.6 | 0.25 | Oui |

**Tableau 6 : Expérience #2 - Décongélation au jour XX**

| **Milieu de Congélation** | **% de viabilité à la décongélation** | **Nombre de cellules par ampoule (*10⁶)** | **Culture** |
|---|---|---|---|
| 67H/33P | 55.2 | 10.4 | Oui |
| | 43.2 | 13.2 | Oui |
| 50H/50P | 60.9 | 28.8 | Oui |
| | 63.7 | 26 | Oui |
| | 70.5 | 12.1 | Oui |
| | 71.7 | 9 | Oui |
| 33H/67P | 73.5 | 12.8 | Oui |
| | 82 | 8.55 | Oui |

**Tableau 7 : Expérience #3 - Décongélation au jour 5**

| **Milieu de Congélation** | **% de viabilité à la décongélation** | **Nombre de cellules par ampoule (*10⁷)** | **Culture** |
|---|---|---|---|
| 10H/90P | nd | nd | nd |
| 20H/80P | 53.4 | 1.10 | Oui |
| 30H/70P | 54.4 | 0.86 | Oui |
| 40H/60P | 53.5 | 1.29 | Oui |
| 50H/50P | 46.3 | 1.00 | Oui |
| 60H/40P | 48 | 1.13 | Oui |
| 70H/30P | nd | nd | nd |
| 80H/20P | 59.2 | 1.14 | Oui |
| 90H/20P | 61.7 | 0.90 | Oui |

**Tableau 8 : Expérience #3 - Décongélation au jour 13**

| **Milieu de Congélation** | **% de viabilité à la décongélation** | **Nombre de cellules par ampoule (*10⁷)** | **Culture** |
|---|---|---|---|
| 10H/90P | 49.2 | 0.94 | Oui |
| 20H/80P | 54.5 | 1.12 | Oui |
| 30H170P | nd | nd | nd |
| 40H/60P | 50.6 | 1.20 | Oui |
| 50H/50P | 58 | 0.73 | Oui |
| 60H/40P | 49.4 | 1.20 | Oui |
| 70H/30P | 54.8 | 0.90 | Oui |
| 80H/20P | 64.7 | 0.99 | Oui |
| 90H/20P | 50 | 0.79 | Oui |

**Tableau 9 : Expérience #4 - Décongélation au jour 8**

| **Milieu de Congélation** | **% de viabilité à la décongélation** | **Nombre de cellules par ampoule (*10⁷)** | **Culture** |
|---|---|---|---|
| 50H/50P | 61.8 | 0.83 | Oui |
| 30H/70P | 68 | 0.75 | Oui |
| 70H/30P | 59.2 | 0.74 | Oui |
| 100P | 21.9 | 0.96 | Non |

**Tableau 10 : Expérience #4 - Décongélation au jour 19**

| **Milieu de Congélation** | **% de viabilité a la décongélation** | **Nombre de cellules par ampoule (*10⁷)** | **Culture** |
|---|---|---|---|
| 50H/50P | 77.3 | 0.66 | Oui |
| 30H/70P | 61.2 | 0.735 | Oui |
| 70H/30P | 62.7 | 0.85 | Oui |
| 100P | 6.5 | 0.69 | Non |

**Tableau 11 : Viabilité et reprise en culture après décongélation à 7 mois.**

| | viabilité | Nbre total de cellules | Nbre de cellules vivantes | Cellules adhérentes 24h | Cellules adhérentes 72h P1 | Cellules adhérentes 120h P2 | Rdt 24h |
|---|---|---|---|---|---|---|---|
| Amp. 1 | 79,8% | 3,13 10⁷ | 2,5 10⁷ | 1,36 10⁷ | | | 54,4% |
| Amp. 2 | 74,5% | 3,1 10⁷ | 2,31 10⁷ | | 1,66 10⁷ | 3,48 10⁷ | |
| Amp. 3 | 79% | 2,72 10⁷ | 2,17 10⁷ | 1,35 10⁷ | | | 62,2% |
| Amp. 4 | 76,6% | 3,25 10⁷ | 2,49 10⁷ | NA | NA | NA | |

**Tableau 13 : Milieux supplémentés de glycérol**

| | V1 | V2 | V3 | Viabilite moyenne |
|---|---|---|---|---|
| HP+10%Glycerol | 41% | 50% | 48,5% | 46,5% |
| HP+5%Glycerol | 70% | 70% | 64% | 68% |
| HP+2.5%Glycerol | 80,5% | 80% | 79% | 79,8% |
| HP+1%Glycerol | 84% | 82% | 87% | 84% |
| | | | | |
| HP | 78% | 75% | 73% | 75% |
| | | | | |
| HP+5% DMSO | 81% | 91% | 93% | 88% |
| HP+2,5%DMSO | 94% | 89% | 92% | 91% |
| HP+1%DMSO | 93% | 92% | 89% | 91% |

**Tableau 14 : Etude de la reprise en culture**

| | Confluence 175cm² | Confluence 275cm² | Confluence 325cm² |
|---|---|---|---|
| HP + 10% Glycerol | pas de repiquage | pas de repiquage | pas de repiquage |
| HP + 5% Glycerol | 4 | 4 | 3,5 |
| HP + 2,5% Glycerol | 4 | 4 | 3,5 |
| HP + 1% Glycerol | 4 | 4 | 3,5 |
| | | | |
| HP | 5 | 5 | 4 |

**Tableau 15 : Essais de congélation programmée en milieu HP+1%Glycérol**

| | Viabilité | conc Cell. / ml | Cell. vivantes totales | Cell. adh 24h | Cell. mortes en susp | rendement |
|---|---|---|---|---|---|---|
| kryosave 1 | 88% | 7,3 10⁵ | 2.19 10⁷ | 1.74 10⁷ | 4.95 10⁶ | 79% |
| C isopropanol 1 | 77% | 6 10⁵ | 1.81 10⁷ | 1.34 10⁷ | 1.32 10⁷ | 74% |
| Kryosave 2 | 83% | 8,4 10⁵ | 2.53 10⁷ | 1.95 10⁷ | 3.3 10⁶ | 77% |
| C isopropanol 2 | 81,5% | 8 10⁵ | 2.41 10⁷ | 1.02 10⁷ | 1.12 10⁷ | 42% |

| | | | | | | |
|---|---|---|---|---|---|---|
| La totalité des cellules de chaque ampoule est mise en culture dans des flacons de 75 2 cm | | | | | | |

**Tableau 16 : Essais de congélation programmée en milieu HP+2,5%Glycérol**

| | Viabilité | conc Cell./ ml | Cell. vivantes totales | Cell. adh 24h | Cell. mortes en susp | rendement |
|---|---|---|---|---|---|---|
| Kryosave 1 | 85% | 1.54 10⁶ | 4.62 10⁷ | 2.07 10⁷ | 5.1 10⁶ | 45% |
| Isopropanol 1 | 82% | 1.12 10⁶ | 3.36 10⁷ | 1.03 10⁷ | 6.9 10⁶ | 30% |
| Kryosave 2 | 78% | 1.76 10⁶ | 5.28 10⁷ | 2.9 10⁷ | 3.9 10⁶ | 55% |
| Isopropanol 2 | 69% | 9.7 105 | 2.91 10⁷ | 1.45 10⁷ | 5.25 10⁶ | 50% |

## Revendications

1. Utilisation d'un milieu comprenant une solution saline, de la gélatine fluide modifiée et de la sérum-albumine humaine pour la congélation de cellules ou de matériel biologique contenant une information génétique autoreproductible ou reproductible dans un système biologique.

2. Utilisation selon la revendication 1, **caractérisée en ce que** la solution saline est une solution saline isotonique avec le plasma.

3. Utilisation selon la revendication 2, **caractérisée en ce que** la solution saline comprend du chlorure de sodium, du chlorure de potassium, du chlorure de magnésium et du lactate de sodium.

4. Utilisation selon la revendication 3, **caractérisée en ce que** la solution saline comprend 2 à 5 g/l de chlorure de sodium, 0.05 à 0.5 g/l de chlorure de potassium, 0.05 à 0.2 g/l de chlorure de magnésium et 0.5 à 4 g/l de lactate de sodium.

5. Utilisation selon la revendication 2, **caractérisée en ce que** la solution saline comprend du chlorure de sodium, du chlorure de potassium, du chlorure de calcium et du lactate de sodium.

6. Utilisation selon la revendication 5, **caractérisée en ce que** la solution saline est du Ringer lactate.

7. Utilisation selon la revendication 6, **caractérisée en ce que** la gélatine fluide modifiée est un produit d'hydrolyse du collagène modifié chimiquement, compatible avec une utilisation pharmaceutique.

8. Utilisation selon la revendication 7, **caractérisée en ce que** la gélatine fluide modifiée a un poids moléculaire moyen compris entre 10 kD et 100 kD et, plus préférentiellement, entre 15 kD et 40 kD.

9. Utilisation selon la revendication 8, **caractérisée en ce que** le produit d'hydrolyse est modifié par réaction avec l'anhydride succinique, citraconique, itaconique, aconitique ou maléique.

10. Utilisation d'un milieu comprenant de la gélatine fluide modifiée 30 g/l, du chlorure de sodium 5.382 g/l, du chlorure de magnésium 0.143 g/l, du chlorure de potassium 0.373 g/l, du lactate de sodium 3.360 g/l et de la sérum-albumine humaine pour la congélation de cellules ou de matériel biologique contenant une information génétique autoreproductible ou reproductible dans un système biologique.

11. Utilisation selon l'une des revendications 1 à 10 **caractérisée en ce que** la sérum-albumine humaine est d'origine plasmatique.

12. Utilisation selon l'une quelconque des revendications précédentes, **caractérisée en ce que** le rapport en poids sérum-albumine humaine/gélatine est compris entre 0.5 et 100.

13. Utilisation selon la revendication 12, **caractérisée en ce que** le rapport en poids sérum-albumine humaine/gélatine est compris entre 0.74 et 60.

14. Utilisation selon la revendication 12, **caractérisée en ce que** le rapport en poids sérum-albumine humaine/gélatine est égal à 0.74, 1.66, 3.3, 6.66, 13.4, 26.66 ou 60.

15. Utilisation selon l'une quelconque des revendications précédentes **caractérisée en ce que** le milieu comprend en outre entre 0.5 et 5% en poids d'un agent de stabilité cellulaire biocompatible, choisi de préférence parmi la glycine, le glycérol, le saccharose ou le glucose.

16. Utilisation selon l'une des revendications 1 à 15, **caractérisée en ce que** le matériel biologique est une population de cellules génétiquement modifiées.

17. Utilisation selon l'une des revendications 1 à 15, **caractérisée en ce que** le matériel biologique est une population de cellules sanguines.

18. Utilisation selon l'une des revendications 1 à 15, **caractérisée en ce que** le matériel biologique est une population de plaquettes.

19. Utilisation selon l'une des revendications 1 à 15, **caractérisée en ce que** le matériel biologique est une population de cellules de la moelle osseuse.

20. Utilisation selon l'une des revendications 1 à 15, **caractérisée en ce que** le matériel biologique est une population de cellules de cultures primaires.

21. Utilisation selon l'une des revendications 1 à 15, **caractérisée en ce que** le matériel biologique est une population de cellules tumorales.

22. Utilisation selon l'une des revendications 1 à 15, **caractérisée en ce que** le matériel biologique est une biopsie de tumeur.

23. Utilisation selon l'une des revendications 1 à 15, **caractérisée en ce que** le matériel biologique est une population de cellules productrices de particules virales.

24. Utilisation selon l'une des revendications 1 à 15, **caractérisée en ce que** le matériel biologique contient des particules virales.

25. Utilisation selon la revendication 24, **caractérisée en ce que** le matériel biologique contient des particules virales recombinantes détectives.

26. Composition comprenant une population de cellules génétiquement modifiées dans lesquelles une séquence d'acides nucléiques d'intérêt thérapeutique a été introduite, par rapport aux mêmes cellules non génétiquement modifiées, et du milieu selon l'une quelconque des revendications 1 à 15.

27. Composition comprenant une population de cellules sanguines et du milieu selon l'une quelconque des revendications 1 à 15 dans lequel le rapport de poids sérum albumine humaine/gélatine fluide modifiée est compris entre 6,66 et 100.

28. Composition comprenant des plaquettes et du milieu selon l'une quelconque des revendications 1 à 15 dans lequel le rapport de poids sérum albumine humaine/gélatine fluide modifiée est compris entre 6,66 et 100.

29. Composition comprenant une population de cellules de la moelle osseuse et du milieu selon l'une quelconque des revendications 1 à 15 dans lequel le rapport de poids sérum albumine humaine/gélatine fluide modifiée est compris entre 6,66 et 100.

30. Composition comprenant des particules virales et du milieu selon l'une quelconque des revendications 1 à 15.

31. Composition selon la revendication 27 **caractérisée en ce que** les particules virales sont des particules virales recombinantes défectives.

32. Composition comprenant une population de cellules productrices de particules virales qui sont des cellules de lignées cellulaires utilisées pour la production de virus recombinants défectifs, et du milieu selon l'une quelconque des revendications 1 à 15.

33. Composition comprenant une population de cellules de cultures primaires et du milieu selon l'une quelconques des revendications 1 à 15.

34. Composition comprenant une population de cellules de cultures tumorales et du milieu selon l'une quelconque des revendications 1 à 15.

35. Composition comprenant une biopsie de tumeur et du milieu selon l'une quelconque des revendications 1 à 15.

36. Composition comprenant une population de cellules génétiquement modifiées et du milieu selon l'une quelconque des revendications 1 à 15, ladite composition étant sous forme congelée, à une température de -4°C à -200°C.

37. Composition comprenant une population de cellules sanguines et du milieu selon l'une quelconque des revendications 1 à 15, ladite composition étant sous forme congelée, à une température de -4°C à -200°C.

38. Composition comprenant des plaquettes et du milieu selon l'une quelconque des revendications 1 à 15, ladite composition étant sous forme congelée, à une température de -4°C à -200°C.

39. Composition comprenant une population de cellules de la moelle osseuse et du milieu selon l'une quelconque des revendications 1 à 15, ladite composition étant sous forme congelée, à une température de -4°C à -200°C.

40. Composition comprenant une population de cellules productrices de particules virales et du milieu selon l'une quelconque des revendications 1 à 15, ladite composition étant sous forme congelée, à une température de -4°C à -200°C.

## Claims

1. Use of a medium composed of a saline solution, modified fluid gelatin and human serum albumin for freezing cells or biological material containing self-reproducible or reproducible genetic information in a biological system.

2. Use according to claim 1, **characterised in that** the saline solution is an isotonic saline solution with plasma.

3. Use according to claim 2, **characterised in that** the saline solution is composed of sodium chloride, potassium chloride, magnesium chloride and sodium lactate.

4. Use according to claim 3, **characterised in that** the saline solution is composed of 2 to 5 g/l of sodium chloride, 0.05 to 0.5 g/l of potassium chloride, 0.05 to 0.2 g/l of magnesium chloride and 0.5 to 4 g/l of sodium lactate.

5. Use according to claim 2, **characterised in that** the saline solution is composed of sodium chloride, potassium chloride, calcium chloride and sodium lactate.

6. Use according to claim 5, **characterised in that** the saline solution is Ringer's lactate.

7. Use according to claim 6, **characterised in that** the modified fluid gelatin is a hydrolysis product of chemically modified collagen compatible with a pharmaceutical use.

8. Use according to claim 7, **characterised in that** the modified fluid gelatin has an average molecular weight in the range of between 10 kD and 100 kD and more preferred between 15 kD and 40 kD,

9. Use according to claim 8, **characterised in that** the hydrolysis product is modified by reacting with succinic, citraconic, itaconic, aconitic or maleic anhydride.

10. Use of a medium composed of 30 g/l of modified fluid gelatin, 5.382 g/l of sodium chloride, 0.143 g/l of magnesium chloride, 0.373 g/l of potassium chloride, 3.360 g/l of sodium lactate and human serum albumin for freezing cells or biological material containing self-reproducible or reproducible genetic information in a biological system.

11. Use according to one of claims I to 10, **characterised in that** the human serum albumin cornes from plasma.

12. Use according to any one of the preceding claims, **characterised in that** the weight ratio of human serum albumin/gelatin lies in the range of between 0.5 to 100.

13. Use according to claims 12, **characterised in that** the weight ratio of human serum albumin/gelatin lies in the range of between 0.74 to 60.

14. Use according to claim 12, **characterised in that** the weight ratio of human serum albumin/gelatin is equal to 0.74, 1.66, 3.3, 6.66, 13.4, 26.66 or 60.

15. Use according to any one of the preceding claims, **characterised in that** the medium is additionally composed of between 0.5 and 5% by weight of a biocompatible cell stabilising agent preferably selected front glycine, glycerol, saccharose or glucose.

16. Use according to one of claims 1 to 15, **characterised in that** the biological material is a population of genetically modified cells.

17. Use according to one of claims 1 to 15, **characterised in that** the biological material is a population of blood cells.

18. Use according to one of claims I to 15, **characterised in that** the biological material is a population of platelets.

19. Use according to one of claims I to 15, **characterised in that** the biological material is a population of bone marrow cells.

20. Use according to one of claims 1 to 15, **characterised in that** the biological material is a population of cells of primary cultures.

21. Use according to one of claims 1 to 15, **characterised in that** the biological material is a population of tumour cells.

22. Use according to one of claims I to 15, **characterised in that** the biological material is a tumour biopsy.

23. Use according to one of claims 1 to 15, **characterised in that** the biological material is a population of viral particle producer cells.

24. Use according to one of claims 1 to 15, **characterised in that** the biological material contains viral particles.

25. Use according to claim 24, **characterised in that** the biological material contains defective recombinant viral particles.

26. Composition comprising a population of genetically modified cells, into which there has been inverted a nucleic acid sequence of therapeutic interest in relation to the same non-genetically modified cells, and the medium according to any one of claims 1 to 15.

27. Composition comprising a population of blood cells and the medium according to any one of claims 1 to 15, in which the weight ratio of human serum albumin/modifîed fluid gelatin lies in the range of between 6.66 and 100.

28. Composition comprising platelets and the medium according to any one of claims 1 to 15, in which the weight ratio of human serum albumin/modified fluid gelatin lies in the range of between 6.66 and 100.

29. Composition comprising a population of bone marrow cells and the medium according to any one of claims 1 to 15, in which the weight ratio of human serum albumin/modified fluid gelatin lies in the range of between 6.66 and 100.

30. Composition comprising viral particles and the medium according to any one of claims 1 to 15.

31. Composition according to claim 27, **characterised in that** the viral particles are defective recombinant viral particles.

32. Composition comprising a population of viral particle producer cells, which are cells of cell lines used for the production, of defective recombinant viruses, and the medium according to any one of claims 1 to 15.

33. Composition comprising a population of cells of primary cultures and the medium according to any one of claims 1 to 15

34. Composition comprising a population of cells of tumour cultures and the medium according to any one of claims 1 to 15.

35. Composition comprising a tumour biopsy and the medium according to any one of claims I to 15.

36. Composition comprising a population of genetically modified cells and the medium according to any one of claims 1 to 15, wherein said composition is in frozen form at a temperature of -4°C to -200°C.

37. Composition comprising a population of blood cells and the medium according to any one of claims I to 15, wherein said composition is in frozen form at a temperature of -4°C to -200°C.

38. Composition comprising platelets and the medium according to any one of claims 1 to 15, wherein said composition is in frozen form at a temperature of -4°C to -200°C.

39. Composition comprising a population of bone marrow cells and the medium according to any one of claims 1 to 15, wherein said composition is in frozen form at a temperature of -4°C to -200°C.

40. Composition comprising a population of viral particle producer cells and the medium according to any one of claims 1 to 15, wherein said composition is in frozen form at a temperature of -4°C to -200°C.

## Patentansprüche

1. Verwendung eines Mediums, das eine Salzlösung, modifizierte flüssige Gelatine und Humanalbumin umfasst, für das Einfrieren von Zellen oder biologischem Material, das eine selbstreproduzierbare oder in einem biologischen System reproduzierbare genetische Information enthält.

2. Verwendung gemäß Anspruch 1, **dadurch gekennzeichnet, dass** die Salzlösung eine zum Plasma isotonische Salzlösung ist.

3. Verwendung gemäß Anspruch 2, **dadurch gekennzeichnet, dass** die Salzlösung Natriumchlorid, Kaliumchlorid, Magnesiumchlorid und Natriumlactat umfasst.

4. Verwendung gemäß Anspruch 3, **dadurch gekennzeichnet, dass** die Salzlösung 2 g/l bis 5 g/l Natriumchlorid, 0,05 g/l bis 0,5 g/l. Kaliumchlorid, 0,05 g/l bis 0,2 g/l Magnesiumchlorid und 0,5 g/l bis 4 g/l Natriumlactat umfasst.

5. Verwendung gemäß Anspruch 2, **dadurch gekennzeichnet, dass** die Salzlösung Natriumchlorid, Kaliumchlorid, Calciumclorid und Natriumlactat umfasst.

6. Verwendung gemäß Anspruch 5, **dadurch gekennzeichnet, dass** die Salzlösung Ringerlactat ist.

7. Verwendung gemäß Anspruch 6, **dadurch gekennzeichnet, dass** die modifizierte flüssige Gelatine ein Hydrolyseprodukt von chemisch modifiziertem Kollagen ist, das mit einer pharmazeutischen Verwendung kompatibel ist.

8. Verwendung gemäß Anspruch 7, **dadurch gekennzeichnet, dass** die modifizierte flüssige Gelatine ein mittleres Molekulargewicht zwischen 10 kD und 100 kD, und noch bevorzugter zwischen 15 kD und 40kD hat.

9. Verwendung gemäß Anspruch 8, **dadurch gekennzeichnet, dass** das Hydrolyseprodukt durch Umsetzen mit Bernsteinsäure-, Citraconsäure-, Itaconsäure-, Aconitsäure- oder Maleisäure-Anhydrid verändert wird.

10. Verwendung eines Mediums, das 30 g/l modifizierte flüssige Gelatine, 5,382 g/l Natriumchlorid, 0,143 g/l Magnesiumchlorid, 0,373 g/l Kaliumchlorid, 3,360 g/l Natriumlactat und Humanalbumin umfasst, für das Einfrieren von Zellen oder biologischem Material, das eine selbstreproduzierbare oder in einem biologischen System reproduzierbare genetische Information enthält.

11. Verwendung gemäß einem der Ansprüche 1 bis 10, **dadurch gekennzeichnet, dass** das Humanalbumin aus Plasma stammt.

12. Verwendung gemäß irgendeinem der vorhergehenden Ansprüche, **dadurch gekennzeichnet, dass** das Gewichtsverhältnis von Humänalbumin zu Gelatine zwischen 0,5 und 100 beträgt.

13. Verwendung gemäß Anspruch 12, **dadurch gekennzeichnet, dass** das Gewichtsverhältnis von Humanalbumin zu Gelatine zwischen 0,74 und 60 beträgt.

14. Verwendung gemäß Anspruch 12, **dadurch gekennzeichnet, dass** das Gewichtsverhältnis von Humanalbumin zu Gelatine gleich 0,74, 1,66, 3,3, 6,66, 13,4, 26, 66 oder 60 ist.

15. Verwendung gemäß irgendeinem der vorhergehenden Ansprüche, **dadurch gekennzeichnet, dass** das Medium ferner zwischen 0,5 Gew.-% und 5 Gew.-% eines biokompatiblen Zellstabilitätsmittels umfasst, das vorzugsweise aus Glycin, Glycerol, Saccharose oder Glukose ausgewählt wird.

16. Verwendung gemäß einem der Ansprüche 1 bis 15, **dadurch gekennzeichnet, dass** das biologische Material eine Population von genetisch veränderten Zellen ist.

17. Verwendung gemäß einem der Ansprüche 1 bis 15, **dadurch gekennzeichnet, dass** das biologische Material eine Population von Blutzellen ist.

18. Verwendung gemäß einem der Ansprüche 1 bis 15, **dadurch gekennzeichnet, dass** das biologische Material eine Population von Blutplättchen ist.

19. Verwendung gemäß einem der Ansprüche 1 bis 15, **dadurch gekennzeichnet, dass** das biologische Material eine Population von Knochenmarkszellen ist.

20. Verwendung gemäß einem der Ansprüche 1 bis 15, **dadurch gekennzeichnet, dass** das biologische Material eine Population von Primärkulturzellen ist.

21. Verwendung gemäß einem der Ansprüche 1 bis 15, **dadurch gekennzeichnet, dass** das biologische Material eine Population von Tumorzellen ist.

22. Verwendung gemäß einem der Ansprüche 1 bis 15, **dadurch gekennzeichnet, dass** das biologische Material ein Tumorbioptat ist.

23. Verwendung gemäß einem der Ansprüche 1 bis 15, **dadurch gekennzeichnet, dass** das biologische Material eine Population von Viruspartikel produzierenden Zellen ist.

24. Verwendung gemäß einem der Ansprüche 1 bis 15, **dadurch gekennzeichnet, dass** das biologische Material Viruspartikel enthält.

25. Verwendung gemäß Anspruch 24, **dadurch gekennzeichnet, dass** das biologische Material defekte rekombinante Viruspartikel enthält.

26. Zusammensetzung, umfassend eine Population von genetisch veränderten Zellen, in die, im Verhältnis zu den gleichen, genetisch nicht veränderten Zellen, eine Sequenz von Nukleinsäuren von therapeutischem Interesse eingeführt wurde und von dem Medium gemäß irgendeinem der Ansprüche 1 bis 15.

27. Zusammensetzung, umfassend eine Population von Blutzellen und von dem Medium gemäß irgendeinem der Ansprüche 1 bis 15, wobei das Gewichtsverhältnis von Humanalbumin zu modifizierter flüssiger Gelatine zwischen 6,66 und 100 beträgt.

28. Zusammensetzung, umfassend Blutplättchen und von dem Medium gemäß irgendeinem der Ansprüche 1 bis 15, wobei das das Gewichtsverhältnis von Humanalbumin zu modifizierter flüssiger Gelatine zwischen 6,66 und 100 beträgt.

29. Zusammensetzung, umfassend eine Population von Knochenmarkszellen und von dem Medium gemäß irgendeinem der Ansprüche 1 bis 15, wobei das Gewichtsverhältnis von Humanalbumin zu modifizierter flüssiger Gelatine zwischen 6,66 und 100 beträgt.

30. Zusammensetzung, umfassend viruspartikel und von dem Medium gemäß irgendeinem der Ansprüche 1 bis 15.

31. Zusammensetzung gemäß Anspruch 27, **dadurch gekennzeichnet, dass** die Viruspartikel defekte rekombinante Viruspartikel sind.

32. Zusammensetzung, umfassend eine Population von Viruspartikel produzierenden Zellen, die Zellen aus Zelllinien sind, die für das Bilden von defekten rekombinanten Viren verwendet werden, und von dem Medium gemäß irgendeinem der Ansprüche 1 bis 15.

33. Zusammensetzung, umfassend eine Zellpopulation aus Primärkulturen und von dem Medium gemäß irgendeinem der Ansprüche 1 bis 15.

34. Zusammensetzung, umfassend eine Zellpopulation aus Tumorkulturen und von dem Medium gemäß irgendeinem der Ansprüche 1 bis 15.

35. Zusammensetzung, umfassend ein Tumorbioptat und von dem Medium gemäß irgendeinem der Ansprüche 1 bis 15.

36. Zusammensetzung, umfassend eine Population von genetisch veränderten Zellen und von dem Medium gemäß irgendeinem der Ansprüche 1 bis 15, wobei die Zusammensetzung in gefrorener Form mit einer Temperatur von -4°C bis -200°C vorliegt.

37. Zusammensetzung, umfassend eine Population von Blutzellen und von dem Medium gemäß irgendeinem der Ansprüche 1 bis 15, wobei die Zusammensetzung in gefrorener Form mit einer Temperatur von -4°C bis -200°C vorliegt.

38. Zusammensetzung, umfassend Blutplättchen und von dem Medium gemäß irgendeinem der Ansprüche 1 bis 15, wobei die Zusammensetzung in gefrorener Form mit einer Temperatur von - 4°C bis 200°C vorliegt.

39. Zusammensetzung, umfassend eine Population von Knochenmarkszellen und von dem Medium gemäß irgendeinem der Ansprüche 1 bis 15, wobei die Zusammensetzung in gefrorener Form mit einer Temperatur von -4°C bis -200°C vorliegt.

40. Zusammensetzung, umfassend eine Population von viruspartikel produzierenden Zellen und von dem Medium gemäß irgendeinem der Ansprüche 1 bis 15, wobei die Zusammensetzung in gefrorener Form mit einer Temperatur von -4°C bis -200°C vorliegt.
